Europäisches Patentamt

**European Patent Office**

Office européen des brevets

(19)

(11) Publication number: **0 218 732**
**B1**

## EUROPEAN PATENT SPECIFICATION

(12)

(45) Date of publication of the patent specification:
**11.07.90**

(21) Application number: **85111728.3**

(22) Date of filing: **17.09.85**

(51) Int. Cl.⁵: **A61K 7/16**

(54) Dentifrices.

(43) Date of publication of application:
**22.04.87 Bulletin 87/17**

(45) Publication of the grant of the patent:
**11.07.90 Bulletin 90/28**

(84) Designated Contracting States:
**AT BE CH DE FR GB IT LI LU NL SE**

(56) References cited:
**EP-A- 0 003 855**
**EP-A- 0 076 563**
**FR-A- 1 576 570**
**US-A- 1 717 723**
**US-A- 4 132 770**
**US-A- 4 223 003**

**CHEMICAL ABSTRACTS, vol. 96, no. 12, March 1982,
page 411, abstract no. 91675v, Columbus, Ohio, US; &
RO - A - 71 000 (INTREPRINDEREA DE MEDICAMENTE,
BUCURESTI) 16-06-1980**
**Handbook of Chemistry & Physics, Ed. 48, R.C. Weast et
al., pages D81-D82**

**The file contains technical information submitted after
the application was filed and not included in this
specification**

(73) Proprietor: **BEECHAM INC., 3 Garret Mountain Plaza,
West Paterson New Jersey 07424(US)**

(72) Inventor: **Stier, Roger E., 265 Washington Avenue,
Clifton, N. J. 07011(US)**
Inventor: **Vidra, James D., RD 1, Box 54-A, Lebanon,
N.J. 08833(US)**
Inventor: **Misek, Bernard, 12 Tara Drive, Pomona, N.
Y. 10970(US)**

(74) Representative: **Russell, Brian John et al, Beecham
Pharmaceuticals Great Burgh Yew Tree Bottom Road,
Epsom Surrey KT18 5XQ(GB)**

ACTORUM AG

## Description

The present invention relates to a dentifrice, and in particular to a dentifrice in which a buffering system is used to maintain an alkaline pH for a predetermined period of brushing time, after which a colour change occurs due to a lowering of the pH.

Dentifrices in which colour changes occur during use have been previously described, but there has always been a problem in controlling and varying the length of time for such a change to occur. By means of the present invention, the length of time over which the pH of the dentifrice is lowered may easily be varied to produce a desired colour change, and this may be achieved by adjusting the concentration of a buffering system.

According to the present invention there is provided a dentifrice composition comprising an abrasive and a dentally acceptable base, characterised in that the composition includes a buffering system which maintains an alkaline pH for a pre-determined period of brushing time, and a colour indicator which, on contact with saliva, causes a colour change in the dentifrice within the user's mouth after a pre-determined period of time has elapsed.

A preferred buffering system in the composition of the invention comprises a combination of sodium phosphate (di-basic) and sodium hydroxide in a ratio sufficient to maintain an alkaline pH for 30-60 seconds of brushing time.

Preferably, two colour indicators are used in combination so that when the user begins brushing, the first colour indicator gives the dentifrice one colour and at the completion of the predetermined period of time for brushing, the second colour indicator gives the dentifrice a different colour in place of the first colour to show the user that he or she has brushed long enough. The second colour indicator is one activated by the lowering of the pH by the saliva of the user.

A preferred pH responsive colour indicator is phenolphthalein, which will show one colour at the beginning of the brushing period and after a predetermined period of brushing time will change colour. The colour change occurs when the pH of the dentifrice is lowered from its initial alkaline pH to the pH of saliva. At that point it shows a different colour to indicate to the user that he or she has brushed a sufficient length of time.

A combination of pH responsive dyes such as phenolphthalein and FD&C® Blue # 1, D&C® Yellow # 10 and the like may be used in which the dye combination responds to a lowering of the alkaline pH by the saliva. Initially during brushing the phenolphthalein colour will show. After a predetermined period of brushing, the secondary colour will appear as the pH is lowered by the saliva in the mouth of the user, thereby replacing the phenolphthalein colour.

The dentifrices according to the present invention preferably contain 10% to 50% humectant, from 10% to 40% deionized water, from 20% to 40% abrasive such as calcium carbonate, dicalcium phosphate, or silica, from 0.5% to 2.0% binders, from 0.1% to 0.3% sweetners, from 0.5% to 5.0% flavourants, from 0.005% colourants, from 0.5% to 2.0% surfactants, from 0.20% to 0.60% sodium hydroxide having an ionic strength and a pH value adapted to yield a final pH for the dentifrice within the range of 10.5-11.5 and from 0.70% to 4.3% of a buffer (all percentages being percentages by weight). The dentifrice composition of the present invention may also contain an effective amount of an anticaries agent such as sodium monofluorophosphate, sodium fluoride or stannous fluoride or the like, alone or in combination. The dentifrice composition may also contain a desensitizing agent and/or an antimicrobial agent. When phenolphthalein is used as the colour indicator, the colour of the dentifrice will change from deep pink to white after 30-60 seconds of brushing. When secondary dyes such as D&C® Yellow # 10 or FD&C® Blue # 1 are also incorporated, the pink colour will disappear and a yellow or blue colour will appear after 30-60 seconds of brushing.

The dentifrice composition of the invention may be in the form of a striped toothpaste. In that case, the core may be white and have a pH of about 11. The coloured stripes, for example blue, are either clear or opaque and contain a suitable pH sensitive dye. The stripes may have a pH of about 7. In this embodiment of the invention the toothpaste initially shows as white in the user's mouth. Upon brushing, the toothpaste turns a colour, for example pink, and after 30-45 seconds turns white again to indicate to the user that he or she has brushed a sufficient length of time. Alternatively, the white core may have a pH of about 11 and a coloured stripe such as a red stripe having a pH of 11 may be combined with the white core. On brushing, the dentifrice turns pink and after 30-45 seconds returns to the white colour as the saliva lowers the pH, as was the case with the white core and blue stripes. When the colour indicator is incorporated into the stripes of a striped gel toothpaste, this may conveniently be done using a dual compartmentalized tube. The colour indicator may also be combined with a second colouring agent if it is desired to have the dentifrice turn a second colour other than white at the completion of the predetermined brushing period.

The surfactant in the composition of the present invention is preferably a low foaming material, such as an alkali metal salt of a fatty alcohol sulphate, alkane sulphonate and/or amine oxide, (and a particularly preferred surfactant is the sodium salt of lauryl alcohol sulphate. The concentration of surfactant is preferably 1.15%.

The following examples illustrate the present invention:

Example 1

A dentifrice according to the present invention was prepared by combining the ingredients set forth below according to known conventional techniques:

| Ingredients | % w/w |
|---|---|
| Glycerine | 30.350 |
| Sodium carboxymethylcellulose | 1.350 |
| Veegum F | 1.000 |
| Deionized water | 24.890 |
| Sodium monofluorophosphate | 0.760 |
| Sodium saccharin | 0.250 |
| Phenolphthalein solution | 1.000 |
| Sodium hydroxide solution | 0.250 |
| Calcium carbonate | 38.000 |
| Flavourants | 1.000 |
| Sodium lauryl sulfate | 1.500 |
| TOTAL | 100.000 |

Example 2

A dentifrice according to the present invention was prepared by combining the ingredients set forth below according to known conventional techniques:

| Ingredients | % w/w |
|---|---|
| Glycerine | 30.350 |
| Sodium carboxymethylcellulose | 1.350 |
| Veegum F | 1.000 |
| Deionized water | 24.890 |
| Sodium monofluorophosphate | 0.760 |
| Sodium saccharin | 0.250 |
| D & C Yellow® #10 dye | 0.005 |
| Phenolphthalein solution | 1.000 |
| Sodium hydroxide solution | 0.250 |
| TOTAL | 100.000 |

Example 3

A striped dentifrice according to the present invention was prepared by combining the ingredients set forth below according to known conventional techniques:

| Ingredients | % w/w |
|---|---|
| Glycerine | 25.830 |
| Sodium carboxymethylcellulose | 1.350 |
| Veegum F | 1.000 |
| Deionized water | 24.890 |
| Sodium monofluorophosphate | 0.760 |
| Sodium saccharin | 0.250 |
| Phenolphthalein solution | 1.000 |
| Sodium hydroxide solution | 0.510 |
| Calcium carbonate | 38.000 |
| Flavourants | 1.000 |
| Sodium lauryl sulfate | 1.150 |
| Dibasic sodium phosphate (anhydrous) | 4.260 |
| TOTAL | 100.000 |

and adding thereto a striping material prepared by combining the ingredients set forth below according to known conventional techniques:

| Ingredients | % w/w |
|---|---|
| Polyethylene glycol 400 | 3.000 |
| Sodium carboxymethyl cellulose | 0.900 |
| Calcium carrageenan | 0.250 |
| Deionized water | 16.840 |
| Sodium monofluorophosphate | 0.760 |
| Sorbitol 70% | 62.000 |
| Sodium benzoate | 0.200 |
| Sodium saccharin | 0.200 |
| Sodium silicate solution | 0.200 |
| Silica | 13.500 |
| Titanium dioxide | 1.000 |
| Sodium lauryl sulfate | 1.150 |
| TOTAL | 100.000 |

The preparation of dentifrices including striped dentifrices is well known in the art. U.S. Patent Nos. 3 966 863, 3,980,767, 4,328,205 and 4,358,437,. describe toothpastes and methods or production thereof which may be utilized for production of the dentifrices according to the present invention.

## Claims

1. A dentifrice composition comprising an abrasive and a dentally acceptable base, and a colour indicator which upon contact with saliva causes a colour change in the dentifrice within the user's mouth after a predetermined period of time has elapsed, characterised in that (a) the composition includes a buffering system which maintains an alkaline pH for a predetermined period of brushing time and (b) the colour indicator is a material showing one colour at alkaline pH and a different colour after the predetermined period of brushing time has elapsed resulting from the lowering of pH by the saliva of the user.

2. A composition according to claim 1 wherein the colour indicator is phenolphthalein.

3. A composition according to claim 1 or claim 2 wherein two colour indicators are incorporated in the composition, the first colour indicator showing its colour at an alkaline pH and the second colour indicator showing its colour after the predetermined period of brushing time has elapsed resulting from lowering of the pH by the saliva of the user.

4. A composition according to claim 3, wherein the two colour indicators are phenolphthalein and D&C Yellow #10®.

5. A composition according to any one of claims 1 to 4 wherein the buffering system is sodium phosphate (dibasic)-sodium hydroxide combination in an amount sufficient to maintain an alkaline pH for 30-60 seconds of brushing time.

6. A composition according to any one of claims 1 to 5 in which is included an anticaries agent selected from sodium monofluorophosphate, sodium fluoride and stannous fluoride.

7. A composition according to any one of claims 1 to 6 wherein the abrasive is calcium carbonate, dicalcium phosphate or silica.

8. A composition according to any one of claims 1 to 7 in the form of a striped toothpaste having a central white core and pastel or coloured stripes that are either clear or opaque, the core having a pH of about 11 and the stripes containing a pH sensitive dye and having a pH of about 7.

9. A composition according to any one of claims 1 to 7 in the form of a striped toothpaste having a white central core and pastel or coloured stripes that are either clear or opaque, the white central core having a pH of about 11 and the stripes containing at least one colour indicator and having a pH of about 11.

## Patentansprüche

1. Eine Zahnpflegezusammensetzung, enthaltend ein Schleifmittel und eine zahnmedizinisch verträgliche Basis, und einen Farbindikator, welcher bei Kontakt mit Speichel im Mund des Benutzers nach einer bestimmten Zeit eine Farbänderung des Zahnpflegemittels bewirkt, dadurch gekennzeichnet, daß (a) die Zusammensetzung ein Puffersystem umfaßt, welches für eine vorbestimmte Dauer des Zähneputzens einen alkalischen pH-Wert aufrechterhält und (b) der Farbindikator ein Material ist, welches bei einem alkalischen pH-Wert eine bestimmte Farbe aufweist und nach der vorbestimmten Zeit des Zähneputzens aufgrund der Herabsetzung des pH-Wertes durch den Speichel des Benutzers eine andere Farbe aufweist.

2. Eine Zusammensetzung nach Anspruch 1, in welcher der Farbindikator Phenolphthalein ist.

3. Eine Zusammensetzung nach Anspruch 1 oder 2, in welcher zwei Farbindikatoren in der Zusammensetzung vorliegen, wobei der erste Farbindikator seine Farbe bei einem alkalischen pH-Wert aufweist und der zweite Farbindikator seine Farbe nach der vorbestimmten Zeit des Zähneputzens zeigt, welche aufgrund der Herabsetzung des pH-Wertes durch den Speichel des Benutzers entsteht.

4. Eine Zusammensetzung nach Anspruch 3, in welcher die beiden Farbindikatoren Phenolphthalein und D&C-Gelb #10® sind.

5. Eine Zusammensetzung nach einem der Ansprüche 1 bis 4, in welcher das Puffersystem eine Natriumphosphat (dibasisches)/Natriumhydroxid-Kombination ist und in einer für die Aufrechterhaltung eines alkalischen pH-Wertes für eine Zeitdauer des Zähneputzens von 30 bis 60 Sekunden geeigneten Menge vorliegt.

6. Eine Zusammensetzung nach einem der Ansprüche 1 bis 5, in welcher ein Antikariesmittel enthalten ist, das ausgewählt ist aus Natriummonofluorphosphat, Natriumfluorid und Zinndifluorid.

7. Eine Zusammensetzung nach einem der Ansprüche 1 bis 6, in welcher das Schleifmittel Calciumcarbonat, Dicalciumphosphat oder Siliciumdioxid ist.

8. Eine Zusammensetzung nach einem der Ansprüche 1 bis 7 in Form einer gestreiften Zahnpaste mit einem zentralen weißen Kern und pastellfarbenen oder farbigen Streifen, die durchscheinend oder matt sein können, wobei der Kern einen pH-Wert von ca. 11 aufweist und die Streifen einen pH-empfindlichen Farbstoff enthalten und einen pH-Wert von ca. 7 aufweisen.

9. Eine Zusammensetzung nach einem der Ansprüche 1 bis 7 in Form einer gestreiften Zahnpaste mit einem zentralen weißen Kern und pastellfarbenen oder farbigen Streifen, welche durchscheinend oder matt sein können, wobei der weiße zentrale Kern einen pH-Wert von ca. 11 aufweist und die Streifen mindestens einen Farbindikator enthalten und einen pH-Wert von ca. 11 aufweisen.

## Revendications

1.- Composition dentifrice comprenant un abrasif et une base acceptable du point de vue dentaire, et un indicateur de couleur qui, en contact de la salive provoque un changement de couleur dans le dentifrice à l'intérieur de la bouche de l'utilisateur après écoulement d'une période de temps prédéterminée, caractérisée en ce que (a) la composition comprend un système tampon qui maintient un pH alcalin pendant une période prédéterminée de brossage et (b) l'indicateur de couleur est une matière présentant une couleur à pH alcalin et une couleur différente après écoulement de la période prédéterminée de brossage, résultant de l'abaissement du pH par la salive de l'utilisateur.

2.- Composition suivant la revendication 1, caractérisée en ce que l'indicateur de couleur est la phénolphtaléine.

3.- Composition suivant la revendication 1 ou la revendication 2, caractérisée en ce que deux indicateurs de couleur sont incorporés dans la composition, le premier indicateur de couleur présentant sa couleur à un pH alcalin et le second indicateur de couleur présentant sa couleur après écoulement de la période prédéterminée de temps de brossage, résultant de l'abaissement du pH par la salive de l'utilisateur.

4.- Composition suivant la revendication 3, caractérisée en ce que les deux indicateurs de couleur sont la phénolphtaléine et le jaune D&C Yellow #10 (dénomination commerciale).

5.- Composition suivant l'une quelconque des revendications 1 à 4, caractérisée en ce que le système tampon est une combinaison de phosphate de sodium (dibasique)-hydroxyde de sodium en une quantité suffisante pour maintenir un pH alcalin pendant une durée de brossage de 30 à 60 secondes.

6.- Composition suivant l'une quelconque des revendications 1 à 5, caractérisée en ce qu'elle comprend un agent anti-carie choisi parmi le monofluorophosphate de sodium, le fluorure de sodium et le fluorure stanneux.

7.- Composition suivant l'une quelconque des revendications 1 à 6, caractérisée en ce que l'abrasif est le carbonate de calcium, le phosphate dicalcique ou la silice.

8.- Composition suivant l'une quelconque des revendications 1 à 7, caractérisée en ce qu'elle est sous forme d'une pâte dentifrice à rayures ayant un noyau blanc central et des bandes pastel ou colorées, claires ou opaques, le noyau ayant un pH d'environ 11 et les bandes contenant un colorant sensible au pH et ayant un pH d'environ 7.

9.- Composition suivant l'une quelconque des revendications 1 à 7, caractérisée en ce qu'elle est sous forme d'une pâte dentifrice à rayures ayant un noyau central blanc et des bandes pastel ou colorées, claires ou opaques, le noyau central ayant un pH d'environ 11 et les bandes contenant au moins un indicateur de couleur et ayant un pH d'environ 11.